# EUROPEAN PATENT APPLICATION

(11) **EP 3 524 621 A1**
(43) Date of publication of application: **14.08.2019**
(21) Application number: 18215704.0
(22) Date of filing: 01.10.2013
(51) Int. Cl.: C07K 16/24, A61K 39/395, A61P 9/00

(54) **TREATING VASCULAR DISEASE AND COMPLICATIONS THEREOF**

(30) Priority: 04.10.2012 US 201261709754 P
(62) Divisional of application: 13843772.8
(71) Applicant: XBiotech, Inc, Vancouver, BC V6E 2E9 (CA)
(72) Inventor: SIMARD, John, Austin, TX 78744 (US)
(74) Representative: Lee, Nicholas John

(57) **Abstract**

Administration of an antibody that specifically binds IL-1α is useful for reducing the chance or severity of a major adverse clinical event occurring in a mammalian subject having received or expected to receive surgical treatment for a stenosed blood vessel, and for reducing the chance of restenosis occurring (or increasing the time until restenosis occurs) in a mammalian subject having received or expected to receive surgical treatment for a stenosed blood vessel.

## Description

### CROSS-REFERENCE TO RELATED APPLICATIONS

The present application claims the priority of U.S. provisional patent application serial number 61/709,754, entitled "Compositions and Methods for Treating Vascular Disease and Complications from the Treatment of Vascular Diseases," and filed on October 4, 2012.

### FIELD OF THE INVENTION

The invention relates generally to the fields of medicine, vascular biology, and immunology. More particularly, the invention relates to the use of agents such as antibodies (Abs) which specifically bind interleukin-1α(IL-1α) to prevent complications associated with vascular disease and its treatment.

### BACKGROUND

Restenosis of coronary arteries following balloon angioplasty and stent placement is one of the major sources of morbidity in interventional cardiology. Restenosis is thought to result from an inflammatory response to injury sustained from the procedure (i.e. balloon angioplasty) and reaction to the stent itself. Indeed, the desire to reduce the rate of restenosis following stent placement has led to the emergence of drug-eluting stents (DES) in recent years. Though studies of sirolimus and paclitaxel-eluting stents in coronary arteries have shown reduced rates of restenosis compared to bare metal stents, more recent meta-analyses have raised concerns for their apparent increased rates of late stent thrombosis and death.

In addition to hindering the process of neointimal hyperplasia fundamental to restenosis, DES have an unwanted propensity to restrict reendothelialization of the stent luminal surface. The resulting perpetually exposed stent surface appears to predispose patients to later thrombus formation coinciding with the cessation of anticoagulant therapy. Since ongoing anticoagulant therapy is not without significant morbidity in itself, other mechanisms to limit neointimal hyperplasia induced by stent deployment are of great interest.

The inflammatory processes that lead to coronary restenosis after revascularization are also involved in loss of vessel patency following intervention in peripheral artery disease. The superficial femoral artery is a particularly frequent site of intervention and restenosis rates are high. The femoro-popliteal system is not always ideally suited for stent placement due to the length of the lesions, abnormal torque associated with lower extremity vessels and the need for anticoagulation. A revascularization procedure can be performed by balloon angioplasty alone, or atherectomy with or without stent placement. Restenosis occurs as a result of the inflammatory response to the revascularization procedure.

### SUMMARY

The invention is based on the discovery that an agent that specifically targets IL-1α can reduce the rate major adverse clinical events (MACE) and restenosis in patients who underwent surgical treatment (e.g., stenting, balloon angioplasty, and atherectomy) on a stenosed vessel.

Accordingly, the invention features a method of reducing the chance or severity of a major adverse clinical event occurring in a mammalian subject having received or expected to receive surgical treatment for a stenosed blood vessel, the method including the step of administering to the subject a pharmaceutical composition comprising a pharmaceutically acceptable carrier and an amount of an IL-1α-targeting agent such as an anti-IL-1α antibody (Ab) effective to reduce the chance or severity of a major adverse clinical event occurring in the subject.

Also within the invention is a method of reducing the chance of restenosis occurring (or increasing the time until restenosis occurs) in a mammalian subject having received or expected to receive surgical treatment for a stenosed blood vessel, the method including the step of administering to the subject a pharmaceutical composition comprising a pharmaceutically acceptable carrier and an amount of an IL-1α-targeting agent such as an anti-IL-1α Ab effective to reduce the chance that the vessel will become restenosed.

In addition, the invention relates to the use of an IL-1α-targeting agent such as an anti-IL-1α Ab to reduce the chance of a major adverse clinical event occurring in a human subject having received or expected to receive surgical treatment for a stenosed blood vessel, and the use of an IL-1α-targeting agent such as an anti-IL-1α Ab to reduce the chance of restenosis occurring in a human subject having received or expected to receive surgical treatment for a stenosed blood vessel.

The IL-1α-targeting agent can be an anti-IL-1α Ab such as a anti-IL-1α monoclonal Ab (mAb). The anti-IL-1α Ab can be an IgG1 such as the mAb designated as MABp1 (see US patent application 13/225,029 filed September 2, 2011 for a description of this antibody) or a mAb that includes one or more complementarity determining regions (CDRs) of MABp1.

The IL-1α-targeting agent can be formulated in a pharmaceutical composition which can be administered to a subject by injection, subcutaneously, intravenously, or intramuscularly. In the method, the dose administered to the patient can be at least 0.5 (e.g., at least 0.5, 1, 2, 2.5, 3, 3.75, 4, or 5) mg/kg of body weight.

Unless otherwise defined, all technical terms used herein have the same meaning as commonly understood by one of ordinary skill in the art to which this invention belongs. Commonly understood definitions of biological terms can be found in Rieger et al., Glossary of Genetics: Classical and Molecular, 5th edition, Springer-Verlag: New York, 1991; and Lewin, Genes V, Oxford University Press: New York, 1994. Commonly understood definitions of medical terms can be found in Stedman's Medical Dictionary, 27th Edition, Lippincott, Williams & Wilkins, 2000.

As used herein, an "antibody" or "Ab" is an immunoglobulin (Ig), a solution of identical or heterogeneous Igs, or a mixture of Igs. An "Ab" can also refer to fragments and engineered versions of Igs such as Fab, Fab', and F(ab')₂ fragments; and scFv's, heteroconjugate Abs, and similar artificial molecules that employ Ig-derived CDRs to impart antigen specificity. A "monoclonal antibody" or "mAb" is an Ab expressed by one clonal B cell line or a population of Ab molecules that contains only one species of an antigen binding site capable of immunoreacting with a particular epitope of a particular antigen. A "polyclonal antibody" or "polyclonal Ab" is a mixture of heterogeneous Abs. Typically, a polyclonal Ab will include myriad different Ab molecules which bind a particular antigen with at least some of the different Abs immunoreacting with a different epitope of the antigen. As used herein, a polyclonal Ab can be a mixture of two or more mAbs.

An "antigen-binding portion" of an Ab is contained within the variable region of the Fab portion of an Ab and is the portion of the Ab that confers antigen specificity to the Ab (*i.e*., typically the three-dimensional pocket formed by the CDRs of the heavy and light chains of the Ab). A "Fab portion" or "Fab region" is the proteolytic fragment of a papain-digested Ig that contains the antigen-binding portion of that Ig. A "non-Fab portion" is that portion of an Ab not within the Fab portion, *e.g*., an "Fc portion" or "Fc region." A "constant region" of an Ab is that portion of the Ab outside of the variable region. Generally encompassed within the constant region is the "effector portion" of an Ab, which is the portion of an Ab that is responsible for binding other immune system components that facilitate the immune response. Thus, for example, the site on an Ab that binds complement components or Fc receptors (not via its antigen-binding portion) is an effector portion of that Ab.

When referring to a protein molecule such as an Ab, "purified" means separated from components that naturally accompany such molecules. Typically, an Ab or protein is purified when it is at least about 10% (*e.g*., 9%, 10%, 20%, 30% 40%, 50%, 60%, 70%, 80%, 90%, 95%, 98%, 99%, 99.9%, and 100%), by weight, free from the non-Ab proteins or other naturally-occurring organic molecules with which it is naturally associated. Purity can be measured by any appropriate method, *e.g*., column chromatography, polyacrylamide gel electrophoresis, or HPLC analysis. A chemically-synthesized protein or other recombinant protein produced in a cell type other than the cell type in which it naturally occurs is "purified."

By "bind", "binds", or "reacts with" is meant that one molecule recognizes and adheres to a particular second molecule in a sample, but does not substantially recognize or adhere to other molecules in the sample. Generally, an Ab that "specifically binds" another molecule has a K_{d} greater than about 10⁵, 10⁶, 10⁷, 10⁸, 10⁹, 10¹⁰, 10¹¹, or 10¹² liters/mole for that other molecule.

A "therapeutically effective amount" is an amount which is capable of producing a medically desirable effect in a treated animal or human (*e.g*., amelioration or prevention of a disease or symptom of a disease, or extension of survivability or lifespan).

Although methods and materials similar or equivalent to those described herein can be used in the practice or testing of the present invention, suitable methods and materials are described below. In addition, the particular embodiments discussed below are illustrative only and not intended to be limiting.

### DETAILED DESCRIPTION

The invention encompasses compositions and methods for preventing or delaying complications in a human subject having received or expected to receive surgically treatment for a stenosed blood vessel. The below described preferred embodiments illustrate adaptation of these compositions and methods. Nonetheless, from the description of these embodiments, other aspects of the invention can be made and/or practiced based on the description provided below.

### General Methodology

Methods involving conventional immunological and molecular biological techniques are described herein. Immunological methods (for example, assays for detection and localization of antigen-Ab complexes, immunoprecipitation, immunoblotting, and the like) are generally known in the art and described in methodology treatises such as Current Protocols in Immunology, Coligan et al., ed., John Wiley & Sons, New York. Techniques of molecular biology are described in detail in treatises such as Molecular Cloning: A Laboratory Manual, 2nd ed., vol. 1-3, Sambrook et al., ed., Cold Spring Harbor Laboratory Press, Cold Spring Harbor, N.Y., 2001; and Current Protocols in Molecular Biology, Ausubel et al., ed., Greene Publishing and Wiley-Interscience, New York. Ab methods are described in Handbook of Therapeutic Abs, Dubel, S., ed., Wiley-VCH, 2007. General methods of medical treatment are described in McPhee and Papadakis, Current Medical Diagnosis and Treatment 2010, 49th Edition, McGraw-Hill Medical, 2010; and Fauci et al., Harrison's Principles of Internal Medicine, 17th Edition, McGraw-Hill Professional, 2008

### Reducing MACEs and Restenosis

The compositions and methods described herein are useful for reducing the chance or severity of a MACE occurring in a mammalian subject having received or expected to receive surgical treatment for a stenosed blood vessel, as well as reducing the chance of restenosis occurring in the subject or increasing the time until restenosis occurs in the subject. The mammalian subject might be any that suffers from a vascular disease including, human beings, dogs, cats, horses, cattle, sheep, goats, and pigs. Human subjects might be male, female, adults, children, or seniors (65 and older). The mammalian subject can be one with peripheral artery disease, coronary artery disease, renal artery disease, Buerger's disease, atherosclerosis, or ischemia. The subject may also be on that is being or has been treated with anti-coagulants, statins, anti-hypertensive agents, cilostazol, and/or pentoxifylline. The surgical treatment for a stenosed blood vessel can include angioplasty, bypass surgery, atherectomy, and/or stenting (with bare metal or drug-eluting stents).

A MACE can include 30-day death, stroke, myocardial infarction/unstable angina, emergent surgical revascularization, significant embolization of the target limb, thrombosis of the target vessel, or worsening of symptoms of chronic limb ischemia. The reduction or increase, if measureable by percent, can be at least 1, 2, 3, 4, 5, 10, 15, 20, 25, 30, 40, 50, 60, 70, 80, or 90%.

### Antibodies and other Agents that Target IL-1α

Any suitable type of Ab or other biologic agent (e.g., a fusion protein including an IL-1α-binding component such as an IL-1 receptor) that specifically binds IL-1α and prevents or delays complications in a human subject having received or expected to receive surgically treatment for a stenosed blood vessel might be used in the invention. For example, the anti-IL-1α Ab used might be a mAb, a polyclonal Ab, a mixture of mAbs, or an Ab fragment or engineered Ab-like molecule such as an scFv. The Ka of the Ab is preferably at least 1 x10⁹ M⁻¹ or greater (e.g., greater than 9 x10¹⁰ M⁻¹, 8 x10¹⁰ M⁻¹, 7 x10¹⁰ M⁻¹, 6 x10¹⁰ M⁻¹, 5 x10¹⁰ M⁻¹, 4 x10¹⁰ M⁻¹, 3 x10¹⁰ M⁻¹, 2 x10¹⁰ M⁻¹, or 1 x10¹⁰ M⁻¹). In a preferred embodiment, the invention utilizes a fully human or TRUE HUMAN mAb that includes (i) an antigen-binding variable region that exhibits very high binding affinity (e.g., at least nano or picomolar) for human IL-1α and (ii) a constant region. The human Ab is preferably an IgG1, although it might be of a different isotype such as IgM, IgA, or IgE, or subclass such as IgG2, IgG3, or IgG4. One example of a particularly useful mAb is MABp1, an IL-1α-specific IgG1 mAb described in U.S. patent application serial number 12/455,458 filed on June 1, 2009. Other useful mAbs are those that include at least one but preferably all the CDRs of MABp1. CDRs may be determined according to known methods such as described in Ofran et al., J. Immunol., 181:6230, 2008; and Antibody Engineering Volume 2, 2d edition, Konterman and Dubel (eds), Springer, 2010.

Because B lymphocytes which express Ig specific for human IL-1α occur naturally in human beings, a presently preferred method for raising mAbs is to first isolate such a B lymphocyte from a subject and then immortalize it so that it can be continuously replicated in culture. Subjects lacking large numbers of naturally occurring B lymphocytes which express Ig specific for human IL-1α may be immunized with one or more human IL-1α antigens to increase the number of such B lymphocytes. Human mAbs are prepared by immortalizing a human Ab secreting cell (e.g., a human plasma cell). See, e.g., U.S. patent no. 4,634,664.

In an exemplary method, one or more (e.g., 5, 10, 25, 50, 100, 1000, or more) human subjects are screened for the presence of such human IL-1α-specific Ab in their blood. Those subjects that express the desired Ab can then be used as B lymphocyte donors. In one possible method, peripheral blood is obtained from a human donor that possesses B lymphocytes that express human IL-1α-specific Ab. Such B lymphocytes are then isolated from the blood sample, e.g., by cells sorting (e.g., fluorescence activated cell sorting, "FACS"; or magnetic bead cell sorting) to select B lymphocytes expressing human IL-1α-specific Ig. These cells can then be immortalized by viral transformation (e.g., using EBV) or by fusion to another immortalized cell such as a human myeloma according to known techniques. The B lymphocytes within this population that express Ig specific for human IL-1α can then be isolated by limiting dilution methods (e.g., cells in wells of a microtiter plate that are positive for Ig specific for human IL-1α are selected and subcultured, and the process repeated until a desired clonal line can be isolated). See, e.g., Goding, Monoclonal Antibodies: Principles and Practice, pp. 59-103, Academic Press, 1986. Those clonal cell lines that express Ig having at least nanomolar or picomolar binding affinities for human IL-1α are preferred. MAbs secreted by these clonal cell lines can be purified from the culture medium or a bodily fluid (e.g., ascites) by conventional Ig purification procedures such as salt cuts, size exclusion, ion exchange separation, and affinity chromatography.

Although immortalized B lymphocytes might be used in *in vitro* cultures to directly produce mAbs, in certain cases it might be desirable to use heterologous expression systems to produce mAbs. See, e.g., the methods described in U.S. patent application number 11/754,899. For example, the genes encoding an mAb specific for human IL-1α might be cloned and introduced into an expression vector (e.g., a plasmid-based expression vector) for expression in a heterologous host cell (e.g., CHO cells, COS cells, myeloma cells, and E. coli cells). Because Igs include heavy (H) and light (L) chains in an H₂L₂ configuration, the genes encoding each may be separately isolated and expressed in different vectors.

Although generally less preferred due to the greater likelihood that a subject will develop an anti-Ab response, chimeric mAbs (e.g., "humanized" mAbs), which are antigen-binding molecules having different portions derived from different animal species (e.g., variable region of a mouse Ig fused to the constant region of a human Ig), might be used in the invention. Such chimeric Abs can be prepared by methods known in the art. See, e.g., Morrison et al., Proc. Nat'l. Acad. Sci. USA, 81:6851, 1984; Neuberger et al., Nature, 312:604, 1984; Takeda et al., Nature, 314:452, 1984. Similarly, Abs can be humanized by methods known in the art. For example, mAbs with a desired binding specificity can be humanized by various vendors or as described in U.S. Pat. Nos. 5,693,762; 5,530,101; or 5,585,089.

The mAbs described herein might be affinity matured to enhance or otherwise alter their binding specificity by known methods such as VH and VL domain shuffling (Marks et al. Bio/Technology 10:779-783, 1992), random mutagenesis of the hypervariable regions (HVRs) and/or framework residues (Barbas et al. Proc Nat. Acad. Sci. USA 91:3809-3813, 1994; Schier et al. Gene 169:147-155, 1995; Yelton et al. J. Immunol. 155:1994-2004, 1995; Jackson et al., J. Immunol. 154(7):3310-9, 1995; and Hawkins et al, J. Mol. Biol. 226:889-896, 1992. Amino acid sequence variants of an Ab may be prepared by introducing appropriate changes into the nucleotide sequence encoding the Ab. In addition, modifications to nucleic acid sequences encoding mAbs might be altered (e.g., without changing the amino acid sequence of the mAb) for enhancing production of the mAb in certain expression systems (e.g., intron elimination and/or codon optimization for a given expression system). The mAbs described herein can also be modified by conjugation to another protein (e.g., another mAb) or non-protein molecule. For example, a mAb might be conjugated to a water soluble polymer such as polyethylene glycol or a carbon nanotube (See, e.g., Kam et al., Proc. Natl. Acad. Sci. USA 102: 11600-11605, 2005). See, U.S. patent application number 11/754,899.

Preferably, to ensure that high titers of human IL-1α-specific mAb can be administered to a subject with minimal adverse effects, the mAb compositions of the invention are at least 0.5, 1, 2, 3, 4, 5, 6, 7, 8, 9, 10, 11, 12, 13, 14, 15, 20, 25, 30, 35, 40, 45, 50, 60, 70, 80, 90, 95, 96, 97, 98, 99, 99.9 or more percent by weight pure (excluding any excipients). The mAb compositions of the invention might include only a single type of mAb (i.e., one produced from a single clonal B lymphocyte line) or might include a mixture of two or more (e.g., 2, 3, 4, 5, 6, 7, 8, 9, 10 or more) different types of mAbs.

While the IL-1α specific Abs described above are preferred for use in the invention, in some cases, other agents that specifically target IL-1α might be used so long as their administration leads to preventing or delaying complications in a human subject having received or expected to receive surgically treatment for a stenosed blood vessel. These other agents might include small organic molecules, aptamers, peptides, and proteins that specifically bind IL-1α.

### Pharmaceutical Compositions and Methods

The anti-IL-1α Ab compositions may be administered to animals or humans in pharmaceutically acceptable carriers (e.g., sterile saline) that are selected on the basis of mode and route of administration and standard pharmaceutical practice. A list of pharmaceutically acceptable carriers, as well as pharmaceutical formulations, can be found in Remington's Pharmaceutical Sciences, a standard text in this field, and in USP/NF. Other substances may be added to the compositions and other steps taken to stabilize and/or preserve the compositions, and/or to facilitate their administration to a subject.

For example, the Ab compositions might be lyophilized (see Draber et al., J. Immunol. Methods. 181:37, 1995; and PCT/US90/01383); dissolved in a solution including sodium and chloride ions; dissolved in a solution including one or more stabilizing agents such as albumin, glucose, maltose, sucrose, sorbitol, polyethylene glycol, and glycine; filtered (e.g., using a 0.45 and/or 0.2 micron filter); contacted with beta-propiolactone; and/or dissolved in a solution including a microbicide (e.g., a detergent, an organic solvent, and a mixture of a detergent and organic solvent.

The Ab compositions may be administered to animals or humans by any suitable technique. Typically, such administration will be parenteral (*e.g*., intravenous, subcutaneous, intramuscular, or intraperitoneal introduction). The compositions may also be administered directly to a target site by, for example, injection. Other methods of delivery, e.g., liposomal delivery or diffusion from a device impregnated with the composition, are known in the art. The composition may be administered in a single bolus, multiple injections, or by continuous infusion (e.g., intravenously or by peritoneal dialysis).

A therapeutically effective amount is an amount which is capable of producing a medically desirable result in a treated animal or human. An effective amount of anti-IL-1α Ab compositions is an amount which shows clinical efficacy in patients as measured by the improvement in one or more of the characteristics described above. As is well known in the medical arts, dosage for any one animal or human depends on many factors, including the subject's size, body surface area, age, the particular composition to be administered, sex, time and route of administration, general health, and other drugs being administered concurrently. Preferred doses range from about 0.2 to 20 (e.g., 0.05, 0.10, 0.15, 0.2, 0.3, 0.4, 0.5, 1, 2, 3, 4, 5, 6, 7, 8, 9, 10, 15, 20, or 40) mg/kg body weight. The dose may be given repeatedly, e.g., hourly, daily, semi-weekly, weekly, bi-weekly, tri-weekly, or monthly. Preferably 2 or more (e.g., 3, 4, 5, 6, 7, 8, 9, 10 or more) doses are given.

### EXAMPLES

Example 1: CV-18C3 is a sterile injectable liquid formulation of MABpl in a stabilizing isotonic buffer.
Example 2: Analysis of a Phase II Open Label, Randomized Study of the Safety, Pharmacokinetics, and Preliminary Efficacy of an Anti-inflammatory Therapeutic Antibody in Reducing the Risk of Restenosis in Patients Undergoing Percutaneous Femoro-popliteal Revascularization.

Trial subjects with vascular disease were randomized to one of two groups. Both groups were scheduled to receive standard of care treatment (e.g., bare metal stenting, balloon angioplasty, and/or atherectomy). The test group was also treated with MABpl (3.75 mg/kg IV at day 0 and weeks 2, 4, and 6; followed by 200 mg subcutaneously every 4 weeks starting at month 2), while the control group did not receive MABp1. Differences in vascular surgical re-intervention rates were analyzed between the two groups. Major adverse cardiovascular events ("MACE": defined as 30-day death, stroke, myocardial infarction/unstable angina, emergent surgical revascularization, significant embolization of the target limb, thrombosis of the target vessel, or worsening of symptoms of chronic limb ischemia/restenosis) were analyzed as well. Of the 43 subjects evaluated, 22 were in the test group and 21 were in the control group.

Despite randomization, the baseline characteristics of both groups were not balanced. Patients in MABpl group had considerably higher risk of restenosis compared to the control (prevalence of diabetes (59% vs. 24%), more frequent atherectomy procedures (41% vs. 29%)), and less improvement in ankle brachial index (ABI) post-procedure (Table 1).

At the 15 week follow-up, 9% (2 of 22) of the MABpl treated patients had experienced a MACE compared to 24% (5 of 21) in the control group (p=0.24). At this time point, patients in the control group were 3 times more likely to experience MACE compared to MABpl group (odds ratio 3.1, 95% CI 0.53 to 18.3).

Patency was evaluated on the basis of clinical symptoms of peripheral vascular disease (claudication), with confirmation of restenosis by angiography. During the first 15 weeks, two patients (9.5%) in the control group had restenosis of their target vessel that required re-intervention. On the other hand, even with the higher baseline risk, all patients receiving MABp1 maintained vascular patency with no restenosis reported during the 15 week post-intervention period.

**Table 1: baseline characteristics of study population**

| | MABp1 Group (n=22) | Control Group (n=21) | p |
|---|---|---|---|
| Age, year | 63±10 | 64±11 | 0.68 |
| Gender, Male | 14 (64%) | 16 (76%) | 0.51 |
| Lesion length, mean±SD (median), cm | 15.1±10.5 (12) | 16.3±15.8 (9) | 0.78 |
| Baseline ABI, mean±SD (median) | 0.71±0.21 (0.70) | 0.65±0.20 (0.64) | 0.35 |
| Post-op ABI, mean±SD (median) | 0.86±0.17 (0.91) | 0.89±0.21 (0.91) | 0.45 |
| ABI Change at Post-op, mean±SD (median) | 0.26±0.31 (0.27) | 0.40±0.28 (0.48) | 0.23 |
| Diabetes | 13 (59%) | 5 (24%) | 0.03 |
| Renal Insufficiency | 3 (14%) | 3 (14%) | 1.00 |
| Current Smoker | 10 (45%) | 9 (43%) | 0.86 |
| Quit smoking <10 years | 5 (23%) | 4 (19%) | 0.76 |
| Procedure | | | |
| Angioplasty | 7 (32%) | 5 (24%) | 0.73 |
| Atherectomy | 1 (5%) | 1 (5%) | 1.00 |
| Stent placement | 9 (41%) | 10 (48%) | 0.76 |
| Angioplasty+Atheroctomy | 8 (36%) | 4 (19%) | 0.31 |
| Angioplasty+Stent | 6 (27%) | 8 (38%) | 0.53 |
| Angioplasty+Atheroctomy+Stent | 2 (9%) | 1 (5%) | 1.00 |
| Atherectomy + any other procedure | 9 (41%) | 6 (29%) | 0.52 |

### Other Embodiments

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

Aspects and features of the present disclosure are set out in the following numbered clauses which contain the subject matter of the claims of the parent application as filed.
1. Use of an anti-IL-1α antibody to reduce the chance of a major adverse clinical event occurring in a human subject having received or expected to receive surgical treatment for a stenosed blood vessel.
2. The use of clause 1, wherein the anti-IL-1α antibody is a monoclonal antibody.
3. The use of clause 2, wherein the monoclonal antibody is an IgG1.
4. The use of clause 2, wherein the monoclonal antibody comprises a complementarity determining region of MABp1.
5. The use of clause 2, wherein the monoclonal antibody is MABp1.
6. Use of an anti-IL-1α antibody to reduce the chance of restenosis occurring in a human subject having received or expected to receive surgical treatment for a stenosed blood vessel.
7. The use of clause 6, wherein the anti-IL-1α antibody is a monoclonal antibody.
8. The use of clause 7, wherein the monoclonal antibody is an IgG1.
9. The use of clause 7, wherein the monoclonal antibody comprises a complementarity determining region of MABp1.
10. The use of clause 7, wherein the monoclonal antibody is MABp1.
11. A method of reducing the chance of a major adverse clinical event occurring in a human subject having received or expected to receive surgical treatment for a stenosed blood vessel, the method comprising the step of administering to the subject a pharmaceutical composition comprising a pharmaceutically acceptable carrier and an amount of an anti-IL-1α antibody effective to reduce the chance of a major adverse clinical event occurring in the subject.
12. A method of reducing the chance of restenosis occurring in a human subject having received or expected to receive surgical treatment for a stenosed blood vessel, the method comprising the step of administering to the subject a pharmaceutical composition comprising a pharmaceutically acceptable carrier and an amount of an anti-IL-la antibody effective to reduce the chance that the vessel will become restenosed.

## Claims

1. A monoclonal antibody which binds human IL-1α with a Ka of at least 1 X 10⁹ M⁻¹ for use in reducing the chance of a major adverse clinical event occurring in a human subject having received or expected to receive surgical treatment for a stenosed blood vessel.

2. The monoclonal antibody for use of claim 1, wherein the monoclonal antibody is a human or humanized monoclonal antibody.

3. The monoclonal antibody for use of claim 2, wherein the monoclonal antibody is an IgG1.

4. The monoclonal antibody for use of claim 2, wherein the monoclonal antibody comprises a complementarity determining region of MABp1/described in US Patent application No. 12/455,458.

5. The monoclonal antibody for use of claim 2, wherein the monoclonal antibody is MABp1/described in US Patent application No. 12/455,458.

6. A monoclonal antibody which binds human IL-1α with a Ka of at least 1 X 10⁹ M⁻¹ for use in reducing the chance of restenosis occurring in a human subject having received or expected to receive surgical treatment for a stenosed blood vessel.

7. The monoclonal antibody for use of claim 6, wherein the anti-IL-1α antibody is a human or humanized monoclonal antibody.

8. The monoclonal antibody for use of claim 7, wherein the monoclonal antibody is an IgG1.

9. The monoclonal antibody for use of claim 7, wherein the monoclonal antibody comprises a complementarity determining region of MABp1/described in US Patent application No. 12/455,458.

10. The monoclonal antibody for use of claim 7, wherein the monoclonal antibody is MABp1/described in US Patent application No. 12/455,458.
